# EUROPEAN PATENT APPLICATION

(11) **EP 4 609 902 A1**
(43) Date of publication of application: **03.09.2025**
(21) Application number: 25154908.5
(22) Date of filing: 30.01.2025
(51) Int. Cl.: A61M 16/08, A61M 16/00

(54) **FLOW SENSORS AND METHODS OF USING THE SAME**

(30) Priority: 28.02.2024 US 202418590761
(71) Applicant: HONEYWELL INTERNATIONAL INC., Charlotte, NC 28202 (US)
(72) Inventor: PINKERTON, Adam, Charlotte, 28202 (US); ECKERMANN, Martin, Charlotte, 28202 (US); HOOVER, William, Charlotte, 28202 (US); SPROESSEL, Marko, Charlotte, 28202 (US); YEE, Daniel, Charlotte, 28202 (US); AVASTHI, Rahul, Charlotte, 28202 (US)
(74) Representative: Haseltine Lake Kempner LLP

(57) **Abstract**

Various embodiments are directed to flow sensors and methods of using the same. In various embodiments, a flow sensor for measuring fluid flow within a breathing system may comprise a housing defining a flow channel and comprising: a housing body and a plurality of coupling members, wherein each coupling member defines an opening through the housing body, the plurality of coupling members comprising: a set of coupling members configured for coupling to a suction tube assembly, each of which is fluidly connected with the housing body via one of a plurality of bottom openings provided on a bottom side of the housing body such that the flow sensor facilitates a contaminant extraction operation defined by a contaminant present within the housing being extracted to the suction tube assembly through the coupling members; wherein each of the coupling members is integrally configured as part of the housing.

## Description

### TECHNICAL FIELD

The present application relates generally to a flow sensor. More specifically, the present application relates generally to a flow sensor that is configured for operation within a breathing circuit.

### BACKGROUND

A flow sensor may be configured to operate within a breathing circuit that directs the flow of a breathing gas to a patient. For example, a flow sensor may be integrated within the breathing circuit to measure breathing gas flow as the breathing circuit is being used to provide respiration assistance to a patient.

The inventors have identified numerous deficiencies and problems with such flow sensors. Through applied effort, ingenuity, and innovation, many of these identified deficiencies and problems have been solved by developing solutions that are structured in accordance with the embodiments of the present disclosure, many examples of which are described in detail herein.

### BRIEF SUMMARY

Various embodiments are directed to a flow sensor and method of using the same. In various embodiments, a flow sensor may comprise A flow sensor for measuring fluid flow within a breathing system, the flow sensor comprising: a housing defining a flow channel configured to direct a flow of a breathing fluid through the flow sensor, the housing comprising: a housing body configured for receiving the breathing fluid within an internal chamber thereof, the internal chamber defining at least a portion of the flow chamber; a plurality of coupling members, wherein each coupling member of the plurality of coupling members defines an opening through the housing body, the plurality of coupling members comprising: a first set of coupling members configured for coupling to a sensor assembly; and a second set of coupling members configured for coupling to a suction tube assembly, each of the second set of coupling members being fluidly connected with the housing body via one of a plurality of bottom openings provided on a bottom side of the housing body; wherein each of the second set of coupling members is configured for coupling to the suction tube assembly such that the flow sensor is configured to facilitate a contaminant extraction operation defined by at least a portion of a contaminant present within the housing being extracted to the suction tube assembly through one or more of the second set of coupling members; wherein each of the second set of coupling members is integrally configured as part of the housing.

In various embodiments, the housing may further comprise one or more reservoirs fluidly connected with the flow channel defined by the housing via a respective one of the plurality of bottom openings provided on the bottom side of the housing body; wherein the flow sensor is configured such that one or more gravitational forces acts on the at least a portion of the contaminant present within the internal chamber in a direction at least partially towards the bottom opening provided at the bottom side the of the respective portion of the sensor housing. In certain embodiments, the one or more reservoirs may comprise a first reservoir fluidly connected to a first internal chamber of a first housing body portion defined by the housing via a first bottom opening of the plurality of bottom openings provided on the bottom side of the housing body, and a second reservoir fluidly connected to a second internal chamber of a second housing body portion defined by the housing via a second bottom opening of the plurality of bottom openings provided on the bottom side of the housing body. Further, in certain embodiments, a first coupling member of the second set of coupling members may extend from the first reservoir, and a second coupling member of the second set of coupling members extends from the second reservoir. Further still, the first coupling member of the second set of coupling members may be fluidly connected to the first reservoir via a first bottom reservoir opening in a first bottom reservoir side of the first reservoir; and wherein the second coupling member of the second set of coupling members is fluidly connected to the second reservoir via a second bottom reservoir opening in a second bottom reservoir side of the second reservoir. In various embodiments, the first coupling member of the second set of coupling members may be configured for coupling to a first suction tube of the suction tube assembly to define at least a portion of a first suction pathway; and wherein the second coupling member of the second set of coupling members is configured for coupling to a second suction tube of the suction tube assembly to define at least a portion of a second suction pathway, wherein the flow sensor is configured to facilitate extraction of a first contaminate from within the first reservoir via the first suction pathway; and wherein the flow sensor is configured to facilitate extraction of a second contaminate from within the second reservoir via the second suction pathway.

In various embodiments, the first reservoir may define a first internal volume and the second reservoir defines a second internal volume, wherein the first internal volume is different than the second internal volume. In certain embodiments, each of the first coupling member and the second coupling member may comprise a luer lock fitting. Further, each of the first coupling member and the second coupling member may comprise a barbed fitting. In various embodiments, the one or more reservoir may be integrally configured as part of the housing. In various embodiments, the flow sensor may further comprise an outer cover embodying an external shell having an internal volume configured to house at least a portion of the plurality of coupling members therein. In various embodiments, the flow sensor may further comprise an integrated suction valve assembly that is selectively configurable between a plurality of arrangements corresponding to a respective plurality of suction operating conditions. In certain embodiments, the integrated suction valve assembly may comprise an actuation mechanism that is integrated into the outer cover.

In various embodiments, the second set of coupling members may comprise three or more coupling members. In certain embodiments, the second set of coupling members may comprise: a first coupling member configured for coupling to a first suction tube of the suction tube assembly to facilitate fluid communication between a first internal chamber defined by a first housing body portion of the housing and the suction tube assembly; a second coupling member configured for coupling to a second suction tube of the suction tube assembly to facilitate fluid communication between the first internal chamber defined by the first housing body portion and the suction tube assembly; a third coupling member configured for coupling to a third suction tube of the suction tube assembly to facilitate fluid communication between a second internal chamber defined by a second housing body portion of the housing and the suction tube assembly; and a fourth coupling member configured for coupling to a fourth suction tube of the suction tube assembly to facilitate fluid communication between the second internal chamber defined by the second housing body portion of the housing and the suction tube assembly. In various embodiments, each of the plurality of coupling members may be defined at least in part by a secure leak-proof connector. In various embodiments, each of the plurality of coupling members may be configured to extend in a respective direction that is at least substantially parallel to a flow direction defined at least in part by central axis of the flow channel defined by the housing.

Further, in various embodiments, each of the plurality of coupling members may be configured to extend in a respective direction that is at least substantially perpendicular to a flow direction defined at least in part by central axis of the flow channel defined by the housing. In various embodiments, the flow sensor may embody a passive flow differential pressure converter. In various embodiments, each of the first set of coupling members may be fluidly connected with the housing body via one of a plurality of top openings provided on a top side of the housing body, the top side being an opposite side of the housing body relative to the bottom side.

### BRIEF DESCRIPTION OF THE DRAWINGS

Reference will now be made to the accompanying drawings, which are not necessarily drawn to scale, and wherein:
FIG. 1A and 1B illustrate an exemplary environment where a device may be used, according to one or more embodiments described herein;
FIG. 2 illustrates an example flow sensor in accordance with at least one example embodiment of the present disclosure;
FIG. 3 provides a schematic illustration of an exemplary flow sensor integrated within a breathing system in accordance with at least one example embodiment of the present disclosure;
FIG. 4 schematically illustrates a flow sensor according to at least one example embodiment of the present disclosure integrated into an exemplary breathing system;
FIG. 5 is a schematic illustration of an exemplary flow sensor comprising an outer cover in accordance with at least one example embodiment of the present disclosure;
FIG. 6 is a schematic illustration of a flow sensor 700 comprising an outer cover in accordance with at least one example embodiment of the present disclosure; and
FIG. 7 is a schematic illustration of a flow sensor 700 in accordance with at least one example embodiment of the present disclosure.

### DETAILED DESCRIPTION

The present disclosure more fully describes various embodiments with reference to the accompanying drawings. It should be understood that some, but not all embodiments are shown and described herein. Indeed, the embodiments may take many different forms, and accordingly this disclosure should not be construed as limited to the embodiments set forth herein. Rather, these embodiments are provided so that this disclosure will satisfy applicable legal requirements. Like numbers refer to like elements throughout.

It should be understood at the outset that although illustrative implementations of one or more aspects are illustrated below, the disclosed assemblies, systems, and methods may be implemented using any number of techniques, whether currently known or not yet in existence. The disclosure should in no way be limited to the illustrative implementations, drawings, and techniques illustrated below, but may be modified within the scope of the appended claims along with their full scope of equivalents. While values for dimensions of various elements are disclosed, the drawings may not be to scale.

The words "example," or "exemplary," when used herein, are intended to mean "serving as an example, instance, or illustration." Any implementation described herein as an "example" or "exemplary embodiment" is not necessarily preferred or advantageous over other implementations.

Flow sensors may be utilized within a breathing system to measure and/or otherwise characterize the flow of one or more fluids (e.g., breathing gas) within a breathing circuit this is configured to direct the flow of gas to and/or from a patient. For example, flow sensors may be integrated into a breathing circuit that is being used in an intubation procedure to provide respiration assistance to a patient. Breathing gas may be supplied to the patient via the breathing circuit and a flow sensor may be integrated within the breathing circuit to measure the flow rate of the breathing gas traveling through the breathing circuit. As described above Applicant has identified various deficiencies associated with breathing circuits. For example, when a patient is invasively intubated such that a tube is passed down through the patient's throat, mucus and/or other substances may flow out of the tubes defining the breathing circuit and into a flow sensor attached thereto, where they may become lodge and/or accumulate over time.

Moreover, in various applications, a humidifier may be leveraged to maintain air flowing through the breathing circuit at a certain temperature and humidity level. The humidified air may condense inside the flow sensor or breathing circuit due to temperature differential between ambient air flowing through the circuit. Contamination, such as mucus, condensation, and/or the like may become lodged within the flow sensor as it flows through the flow sensor, which may cause the flow sensor to fail or otherwise adversely impact the performance of the flow sensor. For example, operation of internal component(s) of the flow sensor may be impaired, resulting in inaccurate flow rate measurement and/or other parameters.

To remove the mucus (and/or other substance) and the condensation, existing solutions require a medical provider to take the breathing circuit apart and clean the flow sensor, which exposes the patient to environmental conditions, and also exposes the medical provider to the medical condition the patient may have. Generally, the medical provider has to use a separate component (e.g., detached from the breathing circuit) to clean out the flow sensor, re-attach the sensor to the breathing circuit, and re-attach the breathing circuit to the patient. Additionally, flooding of the flow sensor can lead to stoppage of therapy, increased operational effort for clinicians, and increase in waste due to discarding flooded flow sensors.

Embodiments of the present disclosure address the above-mentioned challenges and difficulties. Embodiments of the present disclosure provide a flow sensor configured for use with a breathing circuit that is configured to facilitate a more efficient and less intrusive extraction of contamination (e.g., mucus, condensation, and/or the like) from within the flow sensor that does not require taking apart the flow sensor and/or portions of the breathing circuit coupled therewith. Moreover, embodiments of the present disclosure allow for removal of contamination from the sensor while the patient is using the breathing system.

Some embodiments of the present disclosure include a flow sensor having a housing with a plurality of openings arranged about a bottom side thereof, which provide a fluid connection between the internal chamber(s) of the flow sensor housing and a corresponding plurality of coupling members (e.g., suction tube assembly coupling members) configured for coupling to a suction tube assembly of an exemplary breathing system. For example, the suction tube assembly may be configured for being coupled to a suction device (e.g., a suction machine) configured to suck out contamination from the flow sensor via the fluid connections established by the various coupling members of the flow sensor housing that are arranged about the bottom side of the housing. Some embodiments of the present disclosure include a flow sensor with a housing that comprises one or more reservoirs configured to facilitate the collection of contamination therein during operation of the flow sensor (e.g., as fluid flows therethrough) before any need to clean the flow sensor arises, which, in turn, increases the time to failure of the sensor. By providing a configuration that facilitates an in-line contamination extraction from within the internal chamber of the flow sensor without requiring that the flow sensor and/or the breathing circuit be disassembled, embodiments of the present disclosure prevent exposure of the patient to adverse environment conditions, prevents exposure of medical providers to the medical conditions associated with a patient, increases the performance (e.g., time to failure) of the flow sensor, and reduces the amount of resources that would otherwise be utilized to take the breathing circuit apart and clean (or replace) the flow sensor.

Having described example embodiments, the design of the various devices performing various example operations is provided below. The components illustrated in the figures represent components that may or may not be present in various embodiments of the disclosure described herein such that embodiments may include fewer or more components than those shown in the figures while not departing from the scope of the disclosure.

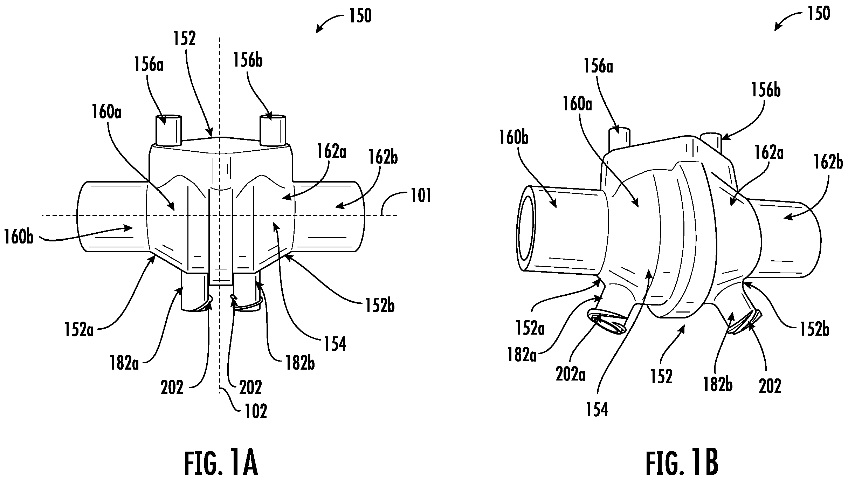

FIGS. 1A and 1B schematically illustrate example flow sensors in accordance with various example embodiments of the present disclosure.

FIG. 1A is a schematic illustration of a flow sensor 150 in accordance with at least one example embodiment of the present disclosure. FIG. 1B is a schematic illustration of a flow sensor 150 in accordance with at least one example embodiment of the present disclosure. In various embodiments, an exemplary flow sensor 150 may be configured for use in a breathing system to measure and/or otherwise characterize one or more fluid (e.g., gas) flows within the breathing system. For example, a breathing system may include a breathing circuit configured to direct the flow of breathing gas to a patient, such as, for example, for use in an intubation procedure to provide respiration assistance to a patient. In various embodiments, an exemplary flow sensor 150 may be integrated into the breathing circuit via an in-line arrangement of the flow sensor 150 relative to one or more components of the breathing circuit in order to measure the flow rate of gas(es) flowing through the one or more breathing tubes.

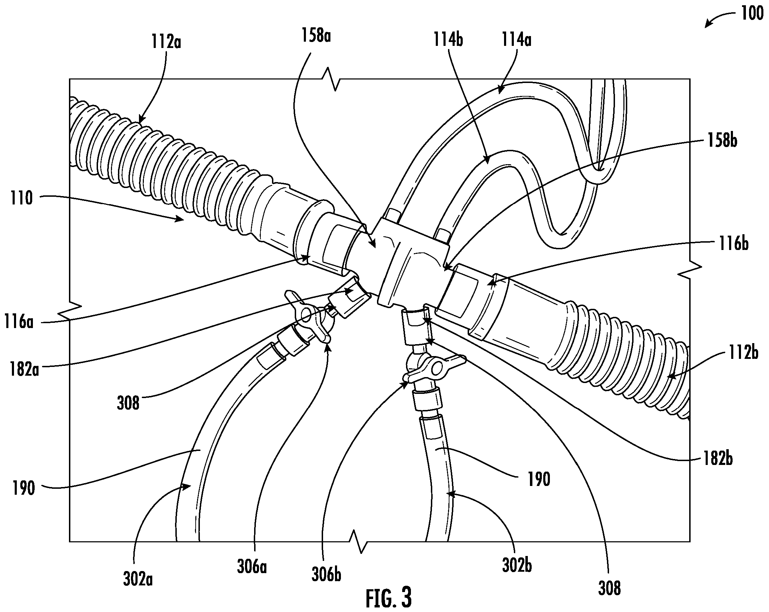

In various embodiments, a flow sensor 150 may be configured for coupling, directly or indirectly, between at least two components of the breathing circuit 110. For example, an exemplary flow sensor 150 may be configured for coupling with one or more breathing tubes defined by the breathing circuit to so as to facilitate an in-line arrangement therewith. As a non-limiting example provided for illustrative purposes, FIG. 3 provides a schematic illustration of an exemplary flow sensor integrated within a breathing system in accordance with at least one example embodiment of the present disclosure. As shown in FIG. 3, the flow sensor 150 is integrated within integrated within a breathing circuit 110 of the breathing system 100 via an in-line arrangement defined by the flow sensor 150 being coupled to at least one of a plurality of breathing tubes 112 of the breathing circuit 110. The breathing circuit 110 may include a plurality of breathing tubes 112 through which gas flows into and out of the flow sensor 150. In various embodiments, as shown in FIG. 3, the flow sensor 150 may comprise a first breathing tube fitting configured for coupling to an end of a first breathing tube 112a of the plurality of breathing tubes 112 (e.g., via an engagement with a first adapter 116a), and a second breathing tube fitting configured for coupling to an end of a second breathing tube 112b of the plurality of breathing tubes 112 (e.g., via a second adapter 116b). For example, one of the first breathing tube 112a or the second breathing tube 112b may be configured for coupling, directly or indirectly, to a ventilator device (e.g., via one or more connectors), while the other of the first breathing tube 112a or second breathing tube 112b may be configured for coupling, directly or indirectly, to a user (e.g., a medical patient) such that the flow sensor 150 is arranged within the breathing circuit 110 to measure at least a portion of the gas flowing between the ventilator device and the user.

Further, as described in further detail herein, an exemplary flow sensor 150 may be configured for coupling, directly or indirectly, with one or more suction tube assemblies to facilitate a fluid connection between one or more suction devices and a respective portion of the flow sensor 150 via each of the one or more suction tube assemblies. For example, the flow sensor 150 may be configured for coupling to a suction tube assembly to facilitate removal of contamination (e.g., mucus, condensation, and/or the like) from within at least a portion of the flow sensor 150 operatively fluidly connected thereto.

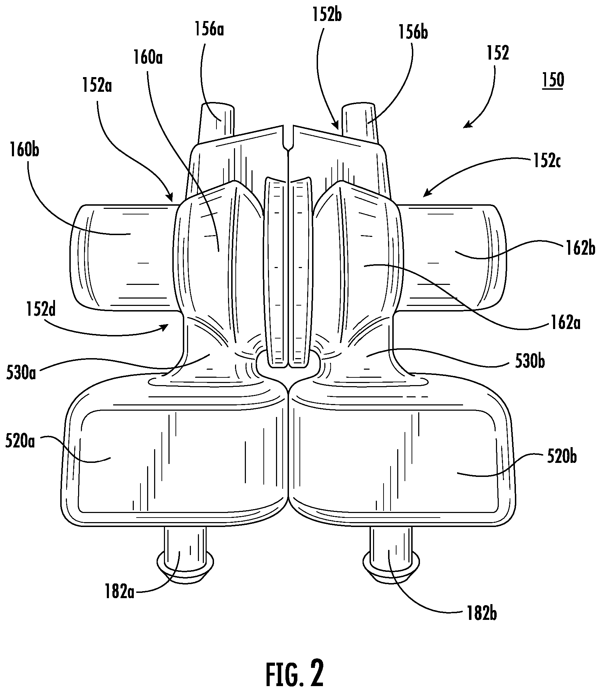

Referring back to the exemplary embodiments illustrated in FIGS. 1A and 1B, an exemplary flow sensor 150 may be a proximal flow sensor based on a differential pressure principle. For example, the flow sensor 150 may describe a passive flow differential pressure converter configured for measuring of gas flow (e.g., breathing gas flow). In various embodiments, the flow sensor 150 may comprise a housing 152 defining a flow channel 154. In various embodiments, the flow channel 154 may define a flow path in a plane that is parallel (e.g., substantially parallel) to a horizontal-axis 101 defined in a first (e.g., horizontal) direction by the housing 152. For example, gas may flow through the flow channel 154 from a first breathing tube (e.g., first breathing tube 112a as illustrated in FIG. 2) to a second breathing tube (e.g., second breathing tube 112b as illustrated in FIG. 2) (or vice versa) along the flow path.

In various embodiments, the housing 152 comprises a first housing 152a and a second housing 152b. In various embodiments, the first housing 152a and the second housing 152b may be coupled together at a respective end portion of the first housing 152a and the second housing 152b. Alternatively and/or additionally, in various embodiments, the housing 152 may embody a singular component such that the first housing 152a and the second housing 152b are defined by respective portions of the same singular material component. In various embodiments, of the first housing 152a and the second housing 152b may each define an internal chamber provided within an interior portion of the respective housing. In various embodiments, the flow sensor 150 may be configured to determine the gas flow (e.g., flow rate) based on the differential pressure between the first housing 152a and the second housing 152b. For example, the differential pressure between the first housing 152a and the second housing 152b may be defined by a differential pressure measured between a first interior chamber of the first housing 152a and a second interior chamber defined by the second housing 152b. In various embodiments, the differential pressure between the first housing 152a and the second housing 152b may be converted to a flow rate corresponding to a volume of breathing gas flowing through the flow sensor 150.

In various embodiments, the first housing 152a and the second housing 152b may each comprise a respective housing body. As illustrated, the first housing 152a comprises a first housing body 158a and the second housing 152b comprises a second housing body 158b. In various embodiments, the first housing body 158a of the first housing 152a may comprise a first body portion 160a and a second body portion 160b. In various embodiments, the second body portion 160b may be defined at least in part by a hollow cylindrical tube having an inner diameter that is smaller than an inner diameter of the adjacent first body portion 160a defined by the same housing body 158a. Further, in various embodiments, the second housing body 158b of the second housing 152b may comprise a first body portion 162a and second body portion 162b. In various embodiments, the second body portion 162b may be defined at least in part by a hollow cylindrical tube having an inner diameter that is smaller than an inner diameter of the adjacent first body portion 162a defined by the same housing body 158b. As described herein, the second body portions 160b, 162b of the first housing body 158a and the second housing body 158b, respectively, may each be configured to receive a breathing tube of an exemplary breathing system to secure at least an end portion of the breathing tube relative to the respective housing portion 152a, 152b of the housing 152 such that the breathing tube is fluidly connected to an interior chamber defined within the respective housing body 158a, 158b.

In various embodiments, the inner diameter of the first body portion 160a of the first housing 152a and the inner diameter of the first body portion 162a of the second housing 152b may be substantially the same. In various embodiments, the inner diameter of the first body portion 160a of the first housing 152a and the inner diameter of the first body portion 162a of the second housing 152b may be different. In various embodiments, the inner diameter of the second body portion 160b of the first housing 152a and the inner diameter of the second body portion 162b of the second housing 152b may be substantially the same. In various embodiments, the inner diameter of the second body portion 160b of the first housing 152a and the inner diameter of the second body portion 162b of the second housing 152b may be different.

The flow sensor 150 may include at least one flow resistance element (not shown) positioned within the housing 152. The at least one flow resistance element may be positioned within the flow sensor 150 in the area where the first housing 152a and second housing 152b meet. For example, a flow resistance element may be positioned substantially parallel to the vertical axis defined by the housing 152 (thus, positioned substantially perpendicular to the horizontal axis defined by the housing 152, which corresponds to the flow path within the flow channel 154 of the housing 152. The flow resistance element may be leveraged to determine the difference in pressure upstream of the flow resistance element and downstream of the flow resistance element. For example, the flow resistance element may be leveraged to determine the difference in pressure within the first housing 152a and the second housing 152b. A flow resistance element may comprise a sheet, a film, or the like, and may be made from a variety of materials (e.g., plastic, composite, and/or the like). In some embodiments, a flow resistance element may be embodied as a flexible flap having a circular shape. In some embodiments, the flow resistance element may comprise a narrow slit. It would be appreciated that in other embodiments, the one or more flow resistance elements may comprise other configurations. For example, in some embodiments, a flow resistance element may have a non-circular shape profile.

In various embodiments, the housing 152 of an exemplary flow sensor 150 may comprise a plurality of coupling members configured for coupling with one or more components of an exemplary breathing system to operably connect at least a portion of the flow sensor to at least a portion of the breathing system. For example, in various embodiments, each coupling member of an exemplary flow sensor 150 may be configured to receive and/or otherwise physically engage a component of a breathing system (e.g., a breathing tube, a sensor tube, a suction tube, and/or the like) to establish both a physical connection in which the component of the breathing system is at least partially secured relative to the flow sensor 150 at the coupling member and a fluid connection between the breathing system component and the flow sensor 150 whereby fluid may be transmitted between the breathing system component and the flow sensor 150 via an opening defined by coupling member. Each coupling member of the plurality of coupling members may define an opening configured to enable a flow of fluid therethrough to facilitate fluid flow into and/or out of a respective portion of the flow sensor 150. In various embodiments, at least a portion of the plurality of coupling members may be configured to facilitate flow measurement. For example, in various embodiments, a first set of coupling members of the plurality of coupling members may be configured for coupling the flow sensor 150 to sensor tube(s), as described herein, which may be coupled to one or more differential pressure measurement devices. As a further example, in various embodiments, a second set of coupling members of the plurality of coupling members may be configured for coupling the flow sensor 150 to one or more suction tube assembly.

In various embodiments, the first housing 152a and the second housing 152b may each comprise at least one coupling member. As shown in FIGS. 1A and 1B, the flow sensor 150 may comprise a plurality of coupling members including a first coupling member 156a defined by the first housing 152a and a second coupling member 156b defined by the second housing 152b. In various embodiments, the first coupling member 156a and the second coupling member 156b may collectively define a first set of coupling members configured for coupling the flow sensor 150 to one or more sensor tube, as described herein. A set of coupling members defined by at least a portion of the plurality of coupling members of the flow sensor 150 may each be provided at the same side of the housing 152, such as, for example, a top side of the housing 152, a bottom side of the housing 152, and/or the like. For example, the plurality of coupling members of an exemplary flow sensor 150 may comprise a first set of coupling members that are each provided at a top side of the housing 152 such that they each extend from a respective housing body (e.g., first housing body 158a and second housing body 158b) and have at least substantially the same configuration as one another. As illustrated in the exemplary embodiments shown in FIGS. 1A and 1B, in an exemplary embodiment wherein the flow sensor 150 comprises a first set of coupling members that includes a first coupling member 156a and the second coupling member 156b that are each configured to facilitate flow measurement via coupling to respective sensor tubes, the first and second coupling members 156a, 156b may each extend from a top side of the housing 152, so as to be provided at a respective top sides of the first housing body 158a and the second housing body 158b, respectively.

In various embodiments, the first housing 152a and the second housing 152b may each comprise a respective plurality of coupling members. For example, in various embodiments, as shown in FIGS. 1A and 1B, an exemplary flow sensor 150 may comprise a plurality of coupling members including a third coupling member 182a defined by the first housing 152a and a fourth coupling member 182b defined by the second housing 152b. In various embodiments, the third coupling member 182a and the fourth coupling member 182b may collectively define a second set of coupling members of the plurality defined by the flow sensor 150 that are each configured for coupling the flow sensor 150 to one or more suction tube assembly, as described herein. For example, in such an exemplary embodiment, the third coupling member 182a and the fourth coupling member 182b may embody a first suction coupling member and a second suction coupling member, respectively, each configured to facilitate removal of contamination from within the respective internal chamber defined by the corresponding housing 152a, 152b. In various embodiments, the plurality of coupling members may include a second set of coupling members comprising three or more suction tube assembly coupling members, each provided at a respective location along a bottom side of the housing 152. As a non-limiting example, in various embodiments, the plurality of coupling members may comprise at least approximately between one suction tube assembly coupling member and six suction tube assembly coupling members (e.g., two suction tube assembly coupling members), each being configured for coupling to a respective portion of the suction tube assembly (e.g., a respective suction tube), as described herein.

As shown in FIGS. 1A and 1B, the third coupling member 182a and the fourth coupling member 182b may extend from the first housing body 158a and the second housing body 158b respectively in one or more directions oriented away from the (e.g., opposite from) the direction(s) in which the first coupling member 156a and second coupling member 156b extend from the first housing body 158a and the second housing body 158b, respectively. For example, the housing 152 may comprise a set of one or more coupling members configured to facilitate flow measurement, and a set of one or more coupling configured to facilitate removal of contamination from the flow sensor, where the sets of one or more coupling members are positioned on opposite sides of the housing 152. In various embodiments, as described herein, the housing 152 of an exemplary flow sensor 150 may comprise a first set of coupling members (e.g., first and second coupling members 156a, 156b) provided at a top side of the housing 152 that is configured to facilitate flow measurement, and a second set of coupling members (e.g., third and fourth coupling members 182a, 182b) provided at an bottom side of the housing 152 (e.g., an opposite side relative to the aforementioned top side) that is configured to facilitate removal of contamination and/or condensate from within the interior portion of the housing 152 of the flow sensor 150.

As described herein, the "top side" of a housing 152 of an exemplary flow sensor 150 may be defined by the one or more surfaces defining a portion of the housing that, upon the flow sensor 150 being integrated into an exemplary breathing circuit (e.g., physically and fluidly connected to one or more breathing tubes in an operable configuration to facilitate measurement of breathing gas flow during patient respiration) is oriented to face in a direction at least substantially away from a ground surface. Further, as described herein, the "bottom side" of a housing 152 of an exemplary flow sensor 150 may be defined by the one or more surfaces defining a portion of the housing that, upon the flow sensor 150 being integrated into an exemplary breathing circuit, as described above, is oriented to face in a direction at least substantially towards a ground surface within the sensor environment. As an illustrative example, upon an exemplary flow sensor 150 being integrated into a breathing circuit, as described herein in reference to the exemplary embodiment illustrated in FIG. 3, at least a portion of the plurality of coupling members defined by the flow sensor 150, such as, for example, a second set of coupling members defined by third and fourth coupling members 182a, 182b, may be provided at a bottom side of the housing 152, each extending away from a respective housing 152a, 152b in a direction at least partially towards a ground surface within the environment of the sensor 150 such that a gravitational force may cause at least a portion of an amount of contaminant flowing through the internal chamber provided within the interior portions of the respective housings 152a, 152b (e.g., including other fluids and/or contaminants present within the internal chambers) to be diverted out of the internal chamber(s) via the coupling members provided at the bottom side of the housing 152. As described herein, the bottom side of the housing 152 may be defined as the portion of the housing 152 that is at least substantially opposite the top side of the housing 152.

In some embodiments, the first coupling member 156a and/or the second coupling member 156b is positioned perpendicular (e.g., substantially perpendicular) with respect to the horizontal-axis 101. It would be appreciated that in some embodiments, the first coupling member 156a and/or the second coupling member 156b may be positioned at an angle with respect to the horizontal-axis 101 defined by the housing 152, where the noted angle is not a perpendicular. In some embodiments, and as shown in FIGS. 1A and 1B, the first coupling member 156a may extend perpendicularly from the first body portion 160a of the first housing 152a and the second coupling member 156b may extend perpendicularly from the first body portion 162a of the second housing 152b. In various embodiments, the first coupling member 156a and the second coupling member 156b have substantially the same diameter. It would be appreciated that in some embodiments, the diameter of the first coupling member 156a and the diameter of the second coupling member 156b may be different.

The third coupling member 182a may extend from the first body portion 160a of the first housing body 158a and the fourth coupling member 182b may extend from the first body portion 162a of the second housing body 158b. As shown in FIG. 1A, in some embodiments, the third coupling member 182a may extend perpendicular from the first housing body 158a (e.g., first body portion 160a thereof) and the fourth coupling member 182b may extend perpendicular from the second housing body 158b (e.g., first body portion 162a thereof). As shown in FIG 1B. in some embodiments, the third coupling member 182a may extend at an angle that is not perpendicular with respect to the first housing body 158a (e.g., first body portion 160a thereof) and the fourth coupling member 182b may extend at an angle that is not perpendicular with respect to the second housing body 158b (e.g., first body portion 162a thereof). As shown in FIG. 1B, each of the third coupling member 182a and fourth coupling member 182b may extend from the first housing body 158a and the second housing body 158b respectively, such that the third coupling member 182a extends away with respect to the vertical axis 102 defined by the housing 152 and the fourth coupling member 182b extends away with respect to the vertical axis 102. It would be appreciated that in some embodiments, each of the third coupling member 182a and fourth coupling member 182b may extend from the first housing body 158a and the second housing body 158b respectively, such that the third coupling member 182a extends towards with respect to the vertical axis 102 defined by the housing 152 and the fourth coupling member 182b extends towards with respect to the vertical axis 102.

Each of the third coupling member 182a and the fourth coupling member 182b may define an opening therethrough (e.g., channel), such that contamination within the flow sensor 150 may be transmitted out of the flow sensor 150 via the third coupling member 182a and the fourth coupling member 182b. In various embodiments, the third coupling member 182a and the fourth coupling member 182b have substantially the same diameter. It would be appreciated that in some embodiments, the diameter of the third coupling member 182a and the diameter of the fourth coupling member 182b may be different.

As described herein, an exemplary breathing system defining a breathing circuit within which the exemplary flow sensor 150 is configured to be operably integrated may include one or more suction tube assembly. In various embodiments, each of the third coupling member 182a and the fourth coupling member 182b may be configured for coupling the flow sensor 150, directly and/or indirectly, to the one or more suction tube assembly to facilitate a fluid connection between one or more suction devices and the interior chambers within the first and second housings 152a, 152b of the flow sensor 150. For example, as described herein, in various embodiments, the third coupling member 182a may be configured for coupling to a first suction tube of a suction tube assembly, such that the first housing 152a is physically and fluidly connected to the first suction tube to define a first suction pathway between the interior chamber within the first housing 152a and the suction tube assembly. Further, in various embodiments, the fourth coupling member 182b may be configured for coupling to a second suction tube of the suction tube assembly, such that the second housing 152b is physically and fluidly connected to the second suction tube to define a second suction pathway between the interior chamber within the second housing 152b and the suction tube assembly.

In various embodiments, a secure leak-free connection configuration is leveraged to couple the third coupling member 182a and the fourth coupling member 182b to the suction tube assembly. In some embodiments, and as shown in FIGS. 1A and 1B, the third coupling member 182a and the fourth coupling member 182b may comprise a luer lock fitting configured to facilitate secure leak-proof connection between each of the third coupling member 182a and the fourth coupling member 182b and the suction tube assembly. For example, and as shown in FIGS 1A and 1B, a portion of the outer surface of each of the third coupling member 182a and fourth coupling member 182b may comprise one or more ridges 202 along the outer surface configured to facilitate a secure leak-proof coupling. Each of the third coupling member 182a and the fourth coupling member 182b may comprise a male luer lock fitting configured to mate with a corresponding female luer lock fitting 308. It would be appreciated that in some embodiments, each of the third coupling member 182a and the fourth coupling member 182b may comprise a female luer lock fitting configured to mate with a corresponding male luer lock fitting. Alternatively, and/or additionally, it would be appreciated that in various embodiments, the third coupling member 182a and the fourth coupling member 182b (e.g., each of the second set of coupling members provided along the bottom side of the housing 152 and configured for coupling to a suction tube assembly) may comprise a secure leak proof coupling configuration that is different from a luer lock configuration, such as, for example, any operable fitting type (e.g., a barbed fitting) configured to facilitate a secure leak-proof connection between the respective coupling member and a corresponding portion of the suction tube assembly.

FIG. 2 illustrates an example flow sensor in accordance with at least one example embodiment of the present disclosure. In particular, FIG. 2 illustrates an exemplary flow sensor 150 comprising at least one reservoir configured for receiving mucus, condensation, and/or other contamination. In various embodiments, the flow sensor 150 may include at least one reservoir fluidly connected to an interior chamber within the housing 152 of the flow sensor 150 and configured to receive at least a portion of a condensate and/or one or more other contaminants from within the internal chambers to be held, stored, captured, and/or otherwise at least temporarily contained within the reservoir. The flow sensor 150 may be configured such that each of the one or more reservoirs are defined by the housing 152 such that the reservoir(s) is integrally configured (e.g., permanently affixed relative to) the housing bodies of the housing 152.

As illustrated, in various embodiments, the housing 152 may comprise a first reservoir 520a and a second reservoir 520b. The first reservoir 520a may be physically and fluidly connected to the first housing 152a via a first intermediate conduit 530a and the second reservoir 520b may be physically and fluidly connected to the first housing 152a via a second intermediate conduit 530b. As illustrated, the first and second intermediate conduits 530a, 530b may each be disposed at a bottom side 152d of the housing 152 (e.g., a portion of the housing 152 opposite the top side 152c), so as to extend from a bottom side of the first and second housings 152a, 152b, respectively. For example, the first reservoir 520a may be disposed opposite the first coupling member 156a and the second reservoir 520b may be disposed opposite the second coupling member 156b. In various embodiments, the first and second reservoirs 520a, 520b may have at least substantially the same (e.g., identical) internal volumes. Further, in various embodiments, the first reservoir 520a connected to the first housing 152a (e.g., via the first intermediate conduit 530a) may define a first internal volume and the second reservoir 520b connected to the second housing 152b (e.g., via the second intermediate conduit 530b) may define a second internal volume that is different than the first internal volume of the first reservoir 520a.

As shown in FIG. 2, the third coupling member 182a and the fourth coupling member 182b may extend from the first reservoir 520a and the second reservoir 520b, respectively, in directions at least partially away from the respective intermediate conduits 530a, 530b. In various embodiments, the third and fourth coupling members 182a, 182b may be provided at a respective bottom side of the first reservoir 520a and the second reservoir 520b, respectively, each extending away from the respective reservoirs 520a, 520b in a direction at least partially towards a ground surface within the environment of the flow sensor 150 such that a gravitational force may facilitate the extraction of at least a portion of the condensate and/or contaminant from within the reservoirs 520a, 520b via the coupling members 182a, 182b. Each of the third coupling member 182a and the fourth coupling member 182b may define an opening therethrough (e.g., channel), such that contamination within the flow sensor 150 include contamination collected in the first reservoir 520a and the second reservoir 520b may be transmitted out of the first reservoir 520a and the second reservoir 520b via the third coupling member 182a and the fourth coupling member 182b respectively.

As shown in FIG. 2. in some embodiments, the third coupling member 182a may extend perpendicular from the first reservoir 520a and the fourth coupling member 182b may extend perpendicular from the second reservoir 520b, such as, for example, in directions at least substantially parallel orientation relative to the first and second intermediate conduits 530a, 530b.

As described above, FIG. 3 schematically illustrates a flow sensor according to at least one example embodiment of the present disclosure integrated into an exemplary breathing system. In particular, FIG. 3 illustrates an exemplary flow sensor operably connected to a suction tube assembly via a set of coupling members that are each integrally configured as part of the housing of the flow sensor and provided at a bottom side of the housing, as described herein. An exemplary suction tube assembly 190 to which the flow sensor 150 is configured to connect to establish an in-line operating configuration may comprise one or more suction tubes. As shown in FIG. 3, an end of a first suction tube 302a of the suction tube assembly 190 may be coupled to the third coupling member 182a defined by the first housing 152a of the flow sensor 150 and an end of second suction tube 302b of the suction tube assembly 190 may be coupled to the fourth coupling member 182b defined by the second housing 152b of the flow sensor 150.

In various embodiments, one or more connectors and/or one or more valves may be leveraged to facilitate the coupling of the third and fourth coupling members 182a, 182b relative to the first suction tube 302a and the second suction tube 302b, respectively. In some embodiments, the one or more connectors comprise luer lock fittings, such as female luer lock fitting 308. In various embodiments, opposing ends of the first suction tube 302a and the second suction tube 302b may be configured for being coupled to at least one suction device configured to generate a fluid flow from one or more of the third and fourth coupling members 182a, 182b towards the at least one suction device. In some embodiments, a first valve 306a of the one or more valves may be disposed between the third coupling member 182a and the first suction tube 302a, and a second valve 306b of the one or more valves may be disposed between the fourth coupling member 182b and the second suction tube 302b.

Further, as illustrated in FIG. 3, in various embodiments, the first coupling member 156a defined by the housing 152 of the exemplary low sensor 150 may be configured for coupling to a first sensor tube 114a such that the first coupling member 156a opens into the first sensor tube 114a. In various embodiments, the second coupling member 156b defined by the housing 152 of the exemplary low sensor 150 may be configured for coupling to a second sensor tube 114b such that the second coupling member 156b opens into the second sensor tube 114b. In various embodiments, a respective opposing end of the first sensor tube 114a and the second sensor tube 114b may be configured for coupling, via one or more connectors, to a measuring unit comprising one or more differential pressure measurement devices (e.g., differential pressure measurement gauges).

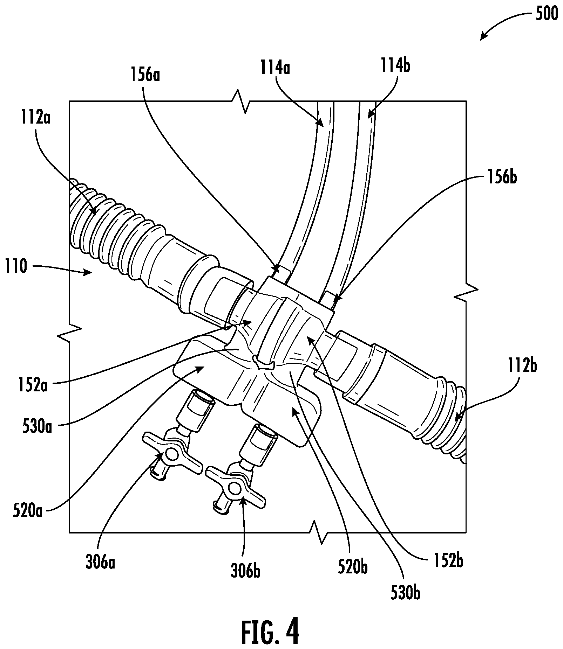

FIG. 4 schematically illustrates a flow sensor according to at least one example embodiment of the present disclosure integrated into an exemplary breathing system. In particular, FIG. 4 illustrates an exemplary flow sensor comprising one or more reservoirs configured to facilitate extraction of various contaminants from within the internal chamber of the sensor housing during operation of the flow sensor. The flow sensor 500 may comprise several of the same components that have been described above in connection to the flow sensor 150. Therefore, a repeated description of the similar component is omitted.

As illustrated, in various embodiments, a first reservoir 520a may be physically and fluidly connected to a first housing 152a of the housing 152 of an exemplary flow sensor 150 via a first intermediate conduit 530a that is configured to extend along an at least substantially linear axis between a bottom side of the first housing 152a, as described herein, and a top side of the first reservoir 520a. Further, in various embodiments, such as, for example, in the exemplary configuration illustrated in FIG. 4, the third coupling member 182a defined by the housing 152 may be configured to extend away from a bottom side of the first reservoir 520a (e.g., a side opposite the top side to which the first intermediate conduit 530a is connected) along an at least substantially linear axis that is oriented in a direction at least substantially parallel to the first intermediate conduit 530a.

Further, in various embodiments, a second reservoir 520b may be physically and fluidly connected to a second housing 152b of the housing 152 of an exemplary flow sensor 150 via a second intermediate conduit 530b that is configured to extend along an at least substantially linear axis between a bottom side of the second housing 152b, as described herein, and a top side of the second reservoir 520b. In various embodiments, such as, for example, in the exemplary configuration illustrated in FIG. 4, the fourth coupling member 182b defined by the housing 152 may be configured to extend away from a bottom side of the second reservoir 520b (e.g., a side opposite the top side to which the second intermediate conduit 530b is connected) along an at least substantially linear axis that is oriented in a direction at least substantially parallel to the second intermediate conduit 530b.

In various embodiments, one or more valves may be disposed between each of a second set of coupling members configured to facilitate removal of contamination and/or condensate from within the interior portion of the housing 152 of the flow sensor 150. For example, as illustrated, in some embodiments, a first valve 306a of the one or more valves may be disposed between the third coupling member 182a and the first suction tube 302a, and a second valve 306b of the one or more valves may be disposed between the fourth coupling member 182b and the second suction tube 302b.

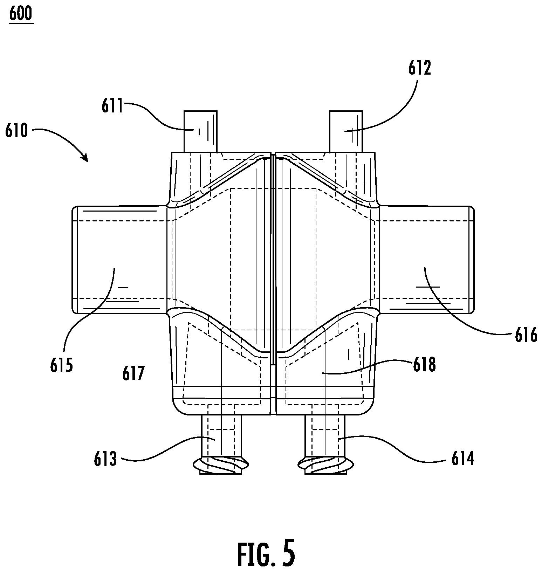

In various embodiments, an exemplary flow sensor may comprise an outer cover embodying an external shell having an internal volume within which the sensor housing (e.g., the first and second housings, the plurality of coupling members, the one or more reservoirs, and/or the like) may be housed. FIG. 5 is a schematic illustration of an exemplary flow sensor comprising an outer cover in accordance with at least one example embodiment of the present disclosure. In particular, the exemplary flow sensor 600 illustrated in FIG. 5 comprises an outer cover 610 configured to house at least a portion of the housing of the flow sensor 600 (e.g., such as, for example, the housing 152 of the exemplary flow sensor 150 embodiments illustrated in FIGS. 1A-4) within an internal volume thereof. The outer cover 610 of the exemplary flow sensor 600 may be configured to facilitate an in-line connection of the flow sensor 600 with one or more components of an exemplary breathing circuit, as described herein, while protecting the components of the flow sensor 600 housing stored within the external shell thereof from various external environmental conditions (e.g., contaminants, forces, and/or the like).

In various embodiments, the outer cover of an exemplary flow sensor 600 may be configured such that the internal volume thereof is defined by a plurality of internal compartments configured to house respective components of the housing of the flow sensor 600 therein. The outer cover 610 of an exemplary flow sensor 600 have a configuration corresponding at least in part to the configuration of the housing of the flow sensor 600, as described herein, such that at least a portion of the housing of the flow sensor 600 may be secured in an operating configuration within the internal volume of the outer cover 610. For example, in various embodiments, the outer cover 610 may be configured such that the internal volume within the external shell is defined by define a plurality of compartments configured for housing one or more of a first and second housings (e.g., including the respective first and second body portions of each), at least a portion of the plurality of coupling members, and one or more reservoir (e.g., including one or more corresponding intermediate conduits connected thereto) defined by the flow sensor housing. As a non-limiting illustrative example, the outer cover 610 of the exemplary flow sensor 600 illustrated in FIG. 5 comprises a first coupling member cover 611 configured for housing a first coupling member defined by the flow sensor housing therein and for connecting with a first sensor tube of an exemplary breathing circuit to operatively couple the first coupling member disposed within the first coupling member cover 611 to the first sensor tube (e.g., by establishing a fluid connection therebetween). Further, the outer cover 610 of the exemplary flow sensor 600 comprises a second coupling member cover 612 configured for housing a second coupling member defined by the flow sensor housing therein and for connecting with a second sensor tube of the exemplary breathing circuit to operatively couple the second coupling member disposed within the second coupling member cover 612 to the second sensor tube (e.g., by establishing a fluid connection therebetween). The first and second coupling member covers 611, 612 defined by the outer cover 610 may be arranged about the outer cover 610 in respective positions and configurations corresponding to the positions and configurations of the first and second coupling members defined by the flow sensor housing (e.g., such as, for example, the first and second coupling members 156a, 156b of the exemplary flow sensor 150 illustrated in FIGS. 1A-4).

As illustrated, in various embodiments, the outer cover 610 of flow sensor 600 may further comprise a first housing body cover 615 configured for storing, housing, and/or otherwise retaining therein a first housing defined by the flow sensor housing (e.g., the first and second body portions thereof, such as, for example, the first and second body portions 160a, 162a of the first housing 152a, as illustrated in the exemplary embodiment of FIGS. 1A and 1B), and for connecting with a first breathing tube of an exemplary breathing circuit to operatively couple the first housing (e.g., the second body portion thereof) disposed within the first housing body cover 615 to the first breathing tube (e.g., by establishing a fluid connection therebetween). In various embodiments, the outer cover 610 of the exemplary flow sensor 600 may further comprise a second housing body cover 616 configured for storing, housing, and/or otherwise retaining therein a second housing defined by the flow sensor housing (e.g., the first and second body portions thereof, such as, for example, the first and second body portions 160b, 162b of the second housing 152b, as illustrated in the exemplary embodiment of FIGS. 1A and 1B), and for connecting with a second breathing tube of an exemplary breathing circuit to operatively couple the second housing (e.g., the second body portion thereof) disposed within the second housing body cover 616 to the second breathing tube (e.g., by establishing a fluid connection therebetween). The first and second housing body covers 615, 616 defined by the outer cover 610 may be arranged about the outer cover 610 in respective positions and configurations corresponding to the positions and configurations of the first and second housings defined by the flow sensor housing (e.g., such as, for example, the first and second housings 152a, 152b of the exemplary flow sensor 150 illustrated in FIGS. 1A and 1B).

Further, as illustrated, the outer cover 610 of the exemplary flow sensor 600 may comprise a third coupling member cover 613 configured for housing a third coupling member defined by the flow sensor housing therein and for connecting with a suction tube assembly (e.g., a first suction tube) of the exemplary breathing circuit to operatively couple the third coupling member disposed within the third coupling member cover 613 to the suction tube assembly (e.g., by establishing a fluid connection therebetween). In various embodiments, the outer cover 610 of the exemplary flow sensor 600 may further comprise a fourth coupling member cover 614 configured for housing a fourth coupling member defined by the flow sensor housing therein and for connecting with the suction tube assembly (e.g., with a second suction tube) of the exemplary breathing circuit to operatively couple the fourth coupling member disposed within the fourth coupling member cover 614 to the suction tube assembly (e.g., by establishing a fluid connection therebetween). The third and fourth coupling member covers 613, 614 defined by the outer cover 610 may be arranged about the outer cover 610 in respective positions and configurations corresponding to the positions and configurations of the third and fourth coupling members defined by the flow sensor housing (e.g., such as, for example, the third and fourth coupling members 182a, 182b of the exemplary flow sensor 150 illustrated in FIGS. 1A-4). For example, as described herein, the third and fourth coupling member covers 613, 614 of the outer cover 610 may be arranged to extend from a bottom side of the outer cover 610.

In various embodiments, as illustrated, the outer cover 610 of an exemplary flow sensor 600 may further comprise first and second reservoir compartments 617, 618 configured for housing therein a first reservoir and a second reservoir defined by the flow sensor housing, respectively. In various embodiments, the first and second reservoir compartments 617, 618 defined by the outer cover 610 may have respective configurations corresponding to the positions and configurations of the first and second reservoirs defined by the flow sensor housing (e.g., such as, for example, the first and second reservoirs 520a, 520b of the exemplary flow sensor 150 illustrated in FIG. 2). For example, as illustrated in FIG. 5, the first and second reservoir compartments 617, 618 of the outer cover 610 may be arranged in between the first housing body cover 615 and the third coupling member cover 613, and the second housing body cover 616 and the fourth coupling member cover 614, respectively.

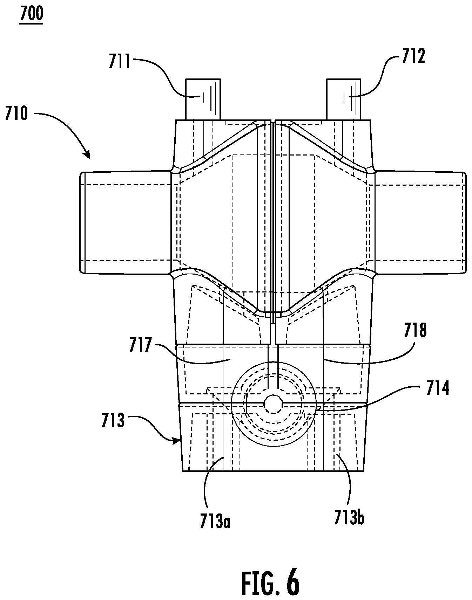

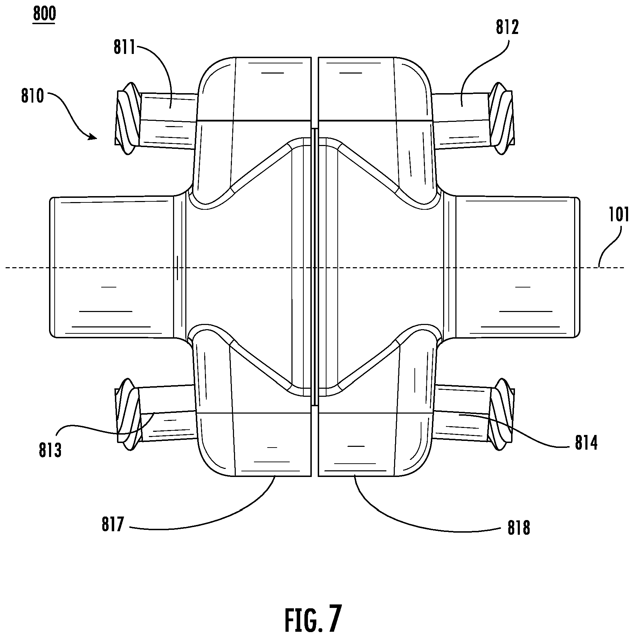

In various embodiments, an exemplary flow sensor may comprise an outer cover configured such that at least a portion of the plurality of coupling members defined by the housing (e.g., the internal housing) of the flow sensor are disposed within the internal volume of the outer cover. As an illustrative example, FIG. 6 is a schematic illustration of a flow sensor 700 comprising an outer cover in accordance with at least one example embodiment of the present disclosure. In particular, the outer cover 710 of the exemplary flow sensor 700 illustrated in FIG. 6 comprises a suction assembly coupling member cover 713 configured to house one or more of the suction tube assembly coupling members defined by the flow sensor housing (e.g., such as, for example, the third and fourth coupling members 182a, 182b of the exemplary flow sensor 150 illustrated in FIGS. 1A-4) such that, upon the suction tube assembly coupling member being coupled to the suction tube assembly, the interface defined therebetween is located within the internal volume of the outer cover 710 (e.g., within the suction assembly coupling member cover 713). For example, in various embodiments, as illustrated, the suction assembly coupling member cover 713 of an outer cover 710 may be provided at a bottom side of the outer cover 710. In various embodiments, the suction assembly coupling member cover 713 may include one or more material recesses, channels, apertures, and/or the like configured to define a fitting within which a portion of an exemplary suction tube assembly (e.g., the end of a suction tube) may be received to facilitate engagement with a respective suction tube assembly coupling member defined by the flow sensor housing for coupling thereto. As a non-limiting example, FIG. 7 illustrates an exemplary embodiment wherein the suction assembly coupling member cover 713 of the outer cover 710 defines a first opening 713a and a second opening 713b provided on a bottom side of the suction assembly coupling member cover 713 and configured for enabling access to respective suction tube assembly coupling members disposed therein to facilitate the coupling of the suction tube assembly with the respective suction tube assembly coupling members at interfaces defined within an internal volume of the outer cover 710.

In various embodiments, the outer cover 710 may comprise an integrated suction valve assembly 714 comprising a button and/or any other suitable valve actuation mechanism that is integrated into the outer cover 710 and selectively actuatable and/or configurable between a plurality of arrangements to selectively establish an open fluid connection between at least a portion of the flow sensor 710 a suction tube assembly coupled thereto. For example, in various embodiments, an integrated suction valve assembly 714 may be configurable in one or more arrangements relative to the suction tube assembly to facilitate selective execution of a suction operation via a suction tube assembly coupled to one or more coupling members of the flow sensor 710. In various embodiments, at least a portion of the integrated suction valve assembly 714 may be accessible from outside the outer cover 710 if the flow sensor 700 such that a user may selectively engage and/or otherwise adjust the integrated suction valve assembly 714 between a plurality of different positions corresponding to varying levels of obstruction along the flow path defined between the reservoirs of the flow sensor 700 and a suction tube assembly coupled to the flow sensor 700 at the one or more suction tube assembly coupling members.

As a non-limiting example, provided for illustrative purposes, in various embodiments, the integrated suction valve assembly 714 may be selectively arranged in a first configuration relative to the outer cover 710 of the flow sensor 700 in which both a first fluid connection between an outlet of a first suction tube assembly coupling member (e.g., first fitting 713a) and a first reservoir (e.g., within the first reservoir compartment 717) and a second fluid connection between an outlet of a second suction tube assembly coupling member (e.g., second fitting 713b) and a second reservoir (e.g., within the second reservoir compartment 718) of the flow sensor 700 are entirely obstructed to prevent any suction from within the flow sensor 700 via either the first or second suction tube assembly coupling members. Further, the integrated suction valve assembly 714 may be selectively arranged in a second configuration relative to the outer cover 710 in which the first fluid connection between the outlet of the first suction tube assembly coupling member (e.g., first fitting 713a) and the first reservoir (e.g., within the first reservoir compartment 717) is entirely obstructed to prevent any suction from within the flow sensor 700 via the first suction tube assembly coupling member, while the second fluid connection between the outlet of the second suction tube assembly coupling member (e.g., second fitting 713b) and the second reservoir (e.g., within the second reservoir compartment 718) of the flow sensor 700 is unobstructed to allow for a suction device to extract a suction fluid flow from within the second reservoir of the flow sensor 700 via the second suction tube assembly coupling member.

Further still, the integrated suction valve assembly 714 may be selectively arranged in a third configuration relative to the outer cover 710 in which the second fluid connection between the outlet of the second suction tube assembly coupling member (e.g., second fitting 713b) and the second reservoir (e.g., within the second reservoir compartment 718) is entirely obstructed to prevent any suction from within the flow sensor 700 via the second suction tube assembly coupling member, while the first fluid connection between the outlet of the first suction tube assembly coupling member (e.g., first fitting 713a) and the first reservoir (e.g., within the first reservoir compartment 717) of the flow sensor 700 is unobstructed to allow for a suction device to extract a suction fluid flow from within the first reservoir of the flow sensor 700 via the first suction tube assembly coupling member. As a further non-limiting example, in various embodiments, the integrated suction valve assembly 714 may be selectively arranged in a fourth configuration relative to the outer cover 710 of the flow sensor 700 in which both a first fluid connection between the outlet of the first suction tube assembly coupling member (e.g., first fitting 713a) and the first reservoir (e.g., within the first reservoir compartment 717) and the second fluid connection between an outlet of the second suction tube assembly coupling member (e.g., second fitting 713b) and the second reservoir (e.g., within the second reservoir compartment 718) of the flow sensor 700 are unobstructed to allow for one or more suction devices to extract respective suction fluid flows from within the first reservoir and the second reservoir of the flow sensor 700 via the first suction tube assembly coupling member and the second suction tube assembly coupling member, respectively. In various embodiments, as illustrated in FIG. 6, the integrated suction valve assembly 714 may comprise one or more rotatable components configured to be rotated relative to the outer cover 710 by a user to selectively adjust the flow sensor 700 between the plurality of configurations corresponding to different suction operating conditions.

In various embodiments, each of the plurality of coupling members defined by the housing of an exemplary flow sensor may be configured to extend from respective portions of the housing bodies in at least substantially parallel directions to facilitate an in-line configuration wherein the flow sensor is configured minimize the physical footprint of the various breathing circuit components (e.g., tubes) coupled therewith. For example, FIG. 7 is a schematic illustration of a flow sensor 700 in accordance with at least one example embodiment of the present disclosure. In particular, the exemplary flow sensor 800 illustrated in FIG. 7 comprises an outer cover 810 that is configured for use with a flow sensor housing having a plurality of coupling members that each extend from a respective surface of the flow sensor housing in a direction that is at least substantially parallel (e.g., within approximately +/- 5 degrees) to the horizontal axis 101 defined by the central axes of the housing body portions at which the flow sensor 800 is configured to connect with first and second breathing tubes to establish an in-line arrangement within an exemplary breathing system.

For example, in various embodiments, the exemplary flow sensor 800 may comprise a first set of coupling members defining sensor tube coupling members configured provided near a top portion of the flow sensor housing, each of which is configured for coupling to respective sensor tubes to facilitate the measurement functionality of the flow sensor 800. In such an exemplary circumstance, the exemplary flow sensor 800 may comprise a first sensor tube coupling member and a second sensor tube coupling member that extend away from respective side surfaces of the flow sensor housing in opposing directions that are both at least substantially parallel to the horizontal axis 101. For example, in such an exemplary configuration, the outer cover 810 of the flow sensor 800 may comprise a first sensor tube coupling member cover 811 and a second sensor tube coupling member cover 812 corresponding to the first and second sensor tube coupling members, respectively, as described herein. As illustrated, the first sensor tube coupling member cover 811 may be configured to extend in a first direction that is oriented at least substantially away from the second sensor tube coupling member cover 812 and defined along a first axis having an at least substantially parallel configuration relative to the horizontal axis 101. Further, in such an exemplary configuration, the second sensor tube coupling member cover 812 may be configured to extend in a second direction that is oriented at least substantially away from the first sensor tube coupling member cover 811 and defined along a second axis having an at least substantially parallel configuration relative to the horizontal axis 101.

Further, in various embodiments, the exemplary flow sensor 800 may comprise a second set of coupling members defining suction tube assembly coupling members configured provided near a top portion of the flow sensor housing, each of which is configured for coupling to a respective portion of a suction tube assembly to facilitate the contaminant extraction functionality of the flow sensor 800, as described herein. In such an exemplary circumstance, the exemplary flow sensor 800 may comprise a first suction tube assembly coupling member and a second suction tube assembly coupling member that extend away from respective side surfaces of the flow sensor housing in opposing directions that are both at least substantially parallel to the horizontal axis 101. For example, in such an exemplary configuration, the outer cover 810 of the flow sensor 800 may comprise a first suction tube assembly coupling member cover 813 and a second suction tube assembly coupling member cover 814 corresponding to the first and second suction tube assembly coupling members of the flow sensor housing, respectively, as described herein. As illustrated, the first suction tube assembly coupling member cover 813 may be configured to extend in a third direction that is oriented at least substantially away from the second suction tube assembly coupling member cover 814 and defined along a third axis having an at least substantially parallel configuration relative to the horizontal axis 101 (e.g., and/or the axis along which the first sensor tube coupling member cover 811 is oriented). Further, in such an exemplary configuration, the second suction tube assembly coupling member cover 814 may be configured to extend in a fourth direction that is oriented at least substantially away from the first suction tube assembly coupling member cover 813 and defined along a fourth axis having an at least substantially parallel configuration relative to the horizontal axis 101 (e.g., and/or the axis along which the second sensor tube coupling member cover 812 is oriented).

Further, in such an exemplary configuration, the second sensor tube coupling member cover 812 may be configured to extend in a second direction that is oriented at least substantially away from the first sensor tube coupling member cover 811 and defined along a second axis having an at least substantially parallel configuration relative to the horizontal axis 101. Alternatively, and/or additionally, in various embodiments, a first set of coupling members comprising a first sensor tube coupling member cover 811 and a second sensor tube coupling member cover 812 may each be configured to extend in respective directions that are oriented in at least substantially the same direction, with both the first and second sensor tube coupling member covers 811, 812 having an at least substantially parallel configuration relative to the horizontal axis 101. Further, in various embodiments, a second set of coupling members comprising a first suction tube coupling member cover 813 and a second suction tube coupling member cover 814 may each be configured to extend in respective directions that are oriented in at least substantially the same direction, with both the first and second suction tube coupling member covers 813, 814 having an at least substantially parallel configuration relative to the horizontal axis 101.

Many modifications and other embodiments will come to mind to one skilled in the art to which this disclosure pertains having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A flow sensor for measuring fluid flow within a breathing system, the flow sensor comprising:
a housing defining a flow channel configured to direct a flow of a breathing fluid through the flow sensor, the housing comprising:
a housing body configured for receiving the breathing fluid within an internal chamber thereof, the internal chamber defining at least a portion of the flow chamber;
a plurality of coupling members, wherein each coupling member of the plurality of coupling members defines an opening through the housing body, the plurality of coupling members comprises:
a first set of coupling members configured for coupling to a sensor assembly; and
a second set of coupling members configured for coupling to a suction tube assembly, each of the second set of coupling members being fluidly connected with the housing body via one of a plurality of bottom openings provided on a bottom side of the housing body;
wherein each of the second set of coupling members is configured for coupling to the suction tube assembly such that the flow sensor is configured to facilitate a contaminant extraction operation defined by at least a portion of a contaminant present within the housing being extracted to the suction tube assembly through one or more of the second set of coupling members;
wherein each of the second set of coupling members is integrally configured as part of the housing.

2. The flow sensor of claim 1, wherein the housing further comprises one or more reservoirs fluidly connected with the flow channel defined by the housing via a respective one of the plurality of bottom openings provided on the bottom side of the housing body; wherein the flow sensor is configured such that one or more gravitational forces acts on the at least a portion of the contaminant present within the internal chamber in a direction at least partially towards the bottom opening provided at the bottom side the of the respective portion of the sensor housing.

3. The flow sensor of claim 2, wherein the one or more reservoirs comprises a first reservoir fluidly connected to a first internal chamber of a first housing body portion defined by the housing via a first bottom opening of the plurality of bottom openings provided on the bottom side of the housing body, and a second reservoir fluidly connected to a second internal chamber of a second housing body portion defined by the housing via a second bottom opening of the plurality of bottom openings provided on the bottom side of the housing body.

4. The flow sensor of claim 3, wherein a first coupling member of the second set of coupling members extends from the first reservoir, and a second coupling member of the second set of coupling members extends from the second reservoir.

5. The flow sensor of claim 4, wherein the first coupling member of the second set of coupling members is fluidly connected to the first reservoir via a first bottom reservoir opening in a first bottom reservoir side of the first reservoir; and wherein the second coupling member of the second set of coupling members is fluidly connected to the second reservoir via a second bottom reservoir opening in a second bottom reservoir side of the second reservoir.

6. The flow sensor of claim 5, wherein the first coupling member of the second set of coupling members is configured for coupling to a first suction tube of the suction tube assembly to define at least a portion of a first suction pathway; and wherein the second coupling member of the second set of coupling members is configured for coupling to a second suction tube of the suction tube assembly to define at least a portion of a second suction pathway, wherein the flow sensor is configured to facilitate extraction of a first contaminate from within the first reservoir via the first suction pathway; and wherein the flow sensor is configured to facilitate extraction of a second contaminate from within the second reservoir via the second suction pathway.

7. The flow sensor of claim 3, wherein the first reservoir defines a first internal volume and the second reservoir defines a second internal volume, wherein the first internal volume is different than the second internal volume.

8. The flow sensor of claim 4, wherein each of the first coupling member and the second coupling member comprises a luer lock fitting.

9. The flow sensor of claim 4, wherein each of the first coupling member and the second coupling member comprises a barbed fitting.

10. The flow sensor of claim 2, wherein the one or more reservoir is integrally configured as part of the housing.

11. The flow sensor of claim 2, further comprising an outer cover embodying an external shell having an internal volume configured to house at least a portion of the plurality of coupling members therein.

12. The flow sensor of claim 11, further comprising an integrated suction valve assembly that is selectively configurable between a plurality of arrangements corresponding to a respective plurality of suction operating conditions.

13. The flow sensor of claim 12, wherein the integrated suction valve assembly comprises an actuation mechanism that is integrated into the outer cover.

14. The flow sensor of claim 1, wherein the second set of coupling members comprises three or more coupling members.

15. The fluid sensor of claim 14, wherein the second set of coupling members comprises:
a first coupling member configured for coupling to a first suction tube of the suction tube assembly to facilitate fluid communication between a first internal chamber defined by a first housing body portion of the housing and the suction tube assembly;
a second coupling member configured for coupling to a second suction tube of the suction tube assembly to facilitate fluid communication between the first internal chamber defined by the first housing body portion and the suction tube assembly;
a third coupling member configured for coupling to a third suction tube of the suction tube assembly to facilitate fluid communication between a second internal chamber defined by a second housing body portion of the housing and the suction tube assembly; and
a fourth coupling member configured for coupling to a fourth suction tube of the suction tube assembly to facilitate fluid communication between the second internal chamber defined by the second housing body portion of the housing and the suction tube assembly.
